(19) Europäisches Patentamt
European Patent Office
Office européen des brevets

(11) **EP 1 452 585 A1**

(12) **EUROPEAN PATENT APPLICATION**
published in accordance with Art. 158(3) EPC

(43) Date of publication:
**01.09.2004 Bulletin 2004/36**

(21) Application number: **02783594.1**

(22) Date of filing: **21.11.2002**

(51) Int Cl.[7]: **C12N 1/16**, C12Q 1/04,
A23L 1/28, A23L 1/305
// C12N1:16, C12R1:85

(86) International application number:
**PCT/JP2002/012200**

(87) International publication number:
**WO 2003/046154 (05.06.2003 Gazette 2003/23)**

(84) Designated Contracting States:
**AT BE BG CH CY CZ DE DK EE ES FI FR GB GR
IE IT LI LU MC NL PT SE SK TR**

(30) Priority: **26.11.2001 JP 2001359781**

(71) Applicant: **Ajinomoto Co., Inc.
Tokyo 104-8315 (JP)**

(72) Inventors:
• **NISHIUCHI, Hiroaki, C/O AJINOMOTO Co., Inc.
Kawasaki-shi, Kanagawa 210-8681 (JP)**
• **SUEHIRO, Mariko, C/O AJINOMOTO Co., Inc.
Kawasaki-shi, Kanagawa 210-8681 (JP)**
• **SUGIMOTO, Reiko, C/O AJINOMOTO Co., Inc.
Tokyo 104-8315 (JP)**
• **NISHIMURA, Yasushi, C/O AJINOMOTO Co., Inc.
Kawasaki-shi, Kanagawa 210-8681 (JP)**
• **KURODA, Motonaka, C/O AJINOMOTO Co., Inc.
Kawasaki-shi, Kanagawa 210-8681 (JP)**

(74) Representative: **Strehl Schübel-Hopf & Partner
Maximilianstrasse 54
80538 München (DE)**

(54) **GAMMA-GLUTAMYLCYSTEINE-PRODUCING YEAST AND METHOD OF SCREENING THE SAME**

(57) A method of screening a yeast strain having a reduced glutathione activity and γ-glutamylcysteine productivity comprising the steps of:

(a) selecting yeast strains having a resistance to a certain concentration range of MNNG from a mass of yeast; and
(b) selecting a strain having a reduced glutathione synthetase activity and γ-glutamylcysteine productivity from among the selected strains.

Fig. 1

Sequence Listing

<110> Ajinomoto Co., Inc.

<120> γ-GLUTAMYLCYSTEINE PRODUCING YEAST AND METHOD FOR SCREENING THE SAME

<130> OP1419

<140>
<141> 2002-11-26

<150> JP 2001-359781
<151> 2001-11-26

<160> 4

<170> PatentIn Ver. 2.0

<210> 1
<211> 20
<212> DNA
<213> Artificial Sequence

<220>
<223> Description of Artificial Sequence: primer for PCR

<400> 1
cagattccga gtttactgga                                         20

<210> 2
<211> 23
<212> DNA
<213> Artificial Sequence

<220>
<223> Description of Artificial Sequence: primer for PCR

<400> 2

agaaggaatg agcctaaaac agc                                                    23

<210> 3
<211> 38
<212> DNA
<213> Artificial Sequence

<220>
<223> Description of Artificial Sequence: primer for PCR

<400> 3

ggcagggaag gcaagtagct ggcattaagt gagccctc                                    38

<210> 4
<211> 38
<212> DNA
<213> Artificial Sequence

<220>
<223> Description of Artificial Sequence: primer for PCR

<400> 4

gagggctcac ttaatgccag ctacttgcct tccctgcc                                    38

**Description**

Technical Field

The present invention relates to a γ-glutamylcysteine-producing yeast strain, to a method of screening it, and to a food utilizing cells of that yeast strain. γ-glutamylcysteine and cysteine produced therefrom are useful in the field of foods.

Background Art

Cysteine is used for the purpose of enhancing the flavor of foods and the like. Known production methods of cysteine include, for example, proteolysis method and a semisynthetic method. The methods that are currently used in the main are the proteolysys method and the semisynthetic method. Although natural food materials having high cysteine contents have been demanded for the purpose of using them to enhance the flavor of foods, such natural food materials have little been known. On the other hand, it has been reported that heat- or enzyme-treatment of yeast extracts containing γ-glutamylcysteine may give rise to food materials having high cysteine contents (WO 00/30474).

γ-glutamylcysteine is synthesized from cysteine and glutamic acid as substrates by the function of γ-glutamyl-cysteine synthetase. On the other hand, glutathione is synthesized from γ-glutamylcysteine and glycine as substrates by the function of glutathione synthetase. It has been reported that a yeast whose glutathione synthetase gene has been disrupted accumulates γ-glutamylcysteine (Ohtake, Y. et al., Agric. Biol. Chem., 54(12), 3145-3150, 1990).

As a method of screening yeast that has lost glutathione synthetase activity, a method using methyl glyoxal sensitivity as a phenotype has been known (Ohtake, Y. et al., Agric. Biol. Chem., 54(12), 3145-3150, 1990). According to the report, 12 strains that cannot produce glutathione were selected out of 300 methyl glyoxal sensitive strains. The report further states that further analysis indicated that eight strains were γ-glutamylcysteine synthetase mutants and the remaining four were glutathione synthetase mutants. In this screening method, one strain out of 75 methyl glyoxal-sensitive strains was a mutant of glutathione synthetase activity. Therefore, at a first glance, the method would seem to be a simple screening method for γ-glutamylcysteine high-accumulating yeasts. However, since no description was made on the number of strains from which the above-mentioned 300 strains of methyl glyoxal-sensitive yeasts were selected, the screening method using methyl glyoxal sensitivity as a phenotypecannot be said to be a simple screening method for high γ-glutamylcysteine-containing yeasts. Furthermore, in the case where methyl glyoxal sensitivity is used as a phenotype, a troublesome operation of replica is required. From this another view point , it cannot be said at all that this is a simple screening method.

Incidentally, a method of utilizing MNNG (N-methyl-N'-nitro-N-nitrosoguanidine) resistance has been known as a method of screening a glutathione-defective strain (Kistler, M. et al., Mutation Research, 173 (1986) 117-120; Kistler, M. et al., Mutagenesis, 5(1) 39-44, 1990). According to this method, all of the obtained yeasts lost γ-glutamyl-cysteine synthetase activity but no strain that has reduced glutathione synthetase activity was obtained. As another method of screening glutathione defective strains, a method of utilizing sodium nitroprusside has been known. Reportedly, in this case too, the obtained yeasts were all γ-glutamylcysteine synthetase mutants (Kistler, M. et al., Mutagenesis, 5(1) 39-44, 1990).

Disclosure of the Invention

Under the above-mentioned background technology, an object of the present invention is to provide yeasts that have a reduced glutathione synthetase activity and that produce γ-glutamylcysteine, and a simple screening method for such yeast strains as well as a γ-glutamylcysteine-containing food utilizing such yeast strains.

Under these circumstances, the inventors of the present invention have made extensive studies and as a result, they have found that resistance to a certain concentration of MNNG used as a phenotype enables efficient selection of strains that have a reduced glutathione synthetase activity and that have γ-glutamylcysteine productivity, thereby achieving the present invention.

That is, the present invention is as follows.

(1) A yeast strain which has a resistanse to MNNG and which produces γ-glutamylcysteine.
(2) A yeast strain according to (1), wherein the strain has a degree of growth of 0.03 or more as calculated by the following formula:

Degree of growth =

$$OD_{660} \text{ of the medium when cultured in MNNG-}$$

$$\text{containing medium / } OD_{660} \text{ of the medium when cultured in}$$

$$\text{MNNG-non-containing medium}$$

when the yeast strain which has been precultured in a liquid medium is collected and inoculated to a YPD medium containing 30 μg/ml of MNNG (MNNG-containing medium) and a YPD medium containing no MNNG (MNNG-non-containing medium) so that the absorbance of each medium at 660 nm ($OD_{660}$) is 0.1, and then the strain is subjected to standing culture at a suitable temperature.

(3) A yeast strain according to (1) or (2), in which the strain has a reduced glutathione synthetase activity.

(4) A yeast strain according to any one of (1) to (3), in which when cultured in an SD medium, the γ-glutamylcysteine content per dry cell in logarithmic phase is 1% by weight or more.

(5) A yeast strain according to any one of (1) to (4), in which the strain has diploidy or more polyploidy.

(6) A yeast strain according to any one of (1) to (5), in which the strain belongs to the genus *Saccharomyces.*

(7) A method of screening a yeast strain having a resistance to MNNG and γ-glutamylcysteine productivity including the steps of:

(a) selecting yeast strains having a resistance to a certain concentration range of MNNG from a mass of yeast; and

(b) selecting a strain having a reduced glutathione synthetase activity and γ-glutamylcysteine productivity from among the selected strains.

(8) A method according to (7), in which in the step (a), the mass of yeast is prepared by subjecting a parent yeast strain to a mutation treatment.

(9) A method according to (7) or (8), in which in the step (a), the yeast strain having a resistance to a certain concentration range of MNNG is selected by using an agar medium on which a concentration gradient of MNNG has been formed.

(10) A method according to any one of (7) to (9), in which in the step (a), a yeast model strain having a reduced glutathione synthetase activity and γ-glutamylcysteine productivity previously isolated is provided and a yeast strain having a resistance to MNNG of the same level as that of the model strain is selected.

(11) A food or beverage including a culture obtained by culturing a yeast strain according to any one of (1) to (6) under a suitable condition, or a fractionation product of the culture containing γ-glutamylcysteine, or the culture or fractionation product in which cysteine has been produced by a heat treatment.

(12) A food or beverage according to (11), in which the food or beverage is an alcoholic beverage, a bread food, or a fermented food flavoring material.

(13) A yeast extract produced by using a culture obtained by culturing a yeast strain according to any one of (1) to (6) under a suitable condition.

(14) A method of producing food which contains γ-glutamylcysteine or cysteine, comprising the steps of culturing a yeast strain according to any one of (1) to (6), mixing the obtained culture or fractionation product thereof, or the culture or fractionation product thereof subjected to heat treatment with a beverage or food raw material, and processing the mixture into a food or beverage.

Hereinafter, the present invention will be described in detail.

The yeast strain of the present invention is a strain that has a resistance to MNNG and that produces γ-glutamylcysteine.

In the present invention, "has a resistance to MNNG" means showing better growth than a wild strain does in the presence of a certain concentration of MNNG. However, it is not required that in the presence of MNNG and in the absence of MNNG, the same degree of growth is shown. In other words, if the growth is better than the growth of the wild strain in the presence of MNNG in spite of bad growth in the presence of MNNG as compared with that in the absence of MNNG, the strain is said to have MNNG resistance. Typically, "having a resistance to MNNG" means that when a yeast strain which has been precultured in a liquid medium is collected and inoculated to a YPD medium containing 30 μg/ml of MNNG (MNNG-containing medium) and to a YPD medium containing no MNNG (MNNG-non-containing medium) so that the absorbance of each medium at 660 nm ($OD_{660}$) is 0.1, and then the strain is subjected to standing culture at a suitable temperature, the degree of growth calculated by the following formula is 0.03 or more, preferably 0.04 or more, more preferably 0.05 or more.

Degree of growth =

OD$_{660}$ of the medium when cultured in MNNG-

containing-medium / OD$_{660}$ of the medium when cultured in

MNNG-non-containing medium

Furthermore, the yeast strain whose degree of growth, as calculated in the same manner as described above except that an MNNG-containing medium was used having the MNNG concentration of 60 μg/ml instead of 30 μg/ml, is 0.015 or more, preferably 0.02 or more, more preferably 0.025 or more is said to have a resistance to MNNG.
Note that too high MNNG resistance indicates high possibility that γ-glutamylcysteine synthetase is deficient, so that it is preferred that the MNNG resistance is not higher than is necessary.
Specifically, for example, when the MNNG concentration is 30 μg/ml, it is desirable that the degree of growth is 1.0 or less, preferably 0.2 or less, more preferably 0.075 or less.
The medium to be used in the above-mentioned preculture includes YPD medium. The preculture may be either shaking culture or standig culture, with shaking culture being preferred. The medium to be used in the main culture includes YPD medium. The main culture may be either shaking culture or standing culture, with standing culture being preferred.
The temperature suitable for the culture may vary depending on the kind of yeast or strain and may be determined by inoculating yeasts onto suitable media, for example, YPD medium, culturing them at various temperatures, and examining a temperature range where growth is good. For example, for *Saccharomyces cerevisiae,* usually a temperature in the vicinity of 30°C is preferable. The culture time is not particularly limited, but it is preferable that the culture is performed in a culture phase that does not exceed the logarithmic growth phase, preferably until the middle logarithmic phase to the late logarithmic phase. Concretely, it is usually about 20 to 40 hours.
In the present invention, "producing γ-glutamylcysteine" refers to accumulating γ-glutamylcysteine in the cells in a larger amount than that of a wild strain of yeast. Preferably, it refers to accumulating 1% or more, more preferably 1.5% or more, γ-glutamylcysteine per dry yeast cell in its logarithmic phase when the yeast cell is cultured in an SD medium, and particularly preferably to accumulating γ-glutamylcysteine with an accumulation amount of glutathione being 0.1% or less when the yeast strain is cultured in an SD medium. The accumulation amount of γ-glutamylcysteine or glutathione refers to the content (%) of γ-glutamylcysteine or glutathione with respect to the solid components of the cell, for example, the weight of cells after heating at 105°C for 4 hours. The term "logarithmic growth phase" refers to a phase in which the number of cells of yeast during culture increases logarithmically with respect to culture time.
The above-mentioned accumulation amount of γ-glutamylcysteine does not have to be maintained throughout all of the logarithmic phase but it is just needed that the above-mentioned value is shown at least at optional points in time in the logarithmic phase, preferably in the following state in the logarithmic growth phase. That is, the above-mentioned state is a logarithmic phase having an absorbance which is not less than 1/2 of the absorbance of the culture broth at a state when the culture is sifted to the stationary phase from logarithmic growth phase.
The compositions of YPD medium and an SD medium are as follows.

| [YPD medium composition] | |
| --- | --- |
| Glucose | 2% |
| Peptone | 1% |
| Yeast extract | 0.5% |
| (pH 5.0) | |

| [SD medium composition] | |
| --- | --- |
| Glucose | 2% |
| Nitrogen Base | 1-fold concentration |

(10-Fold concentration Nitrogen Base is a mixture of 1.7 g of Bacto Yeast Nitrogen Base w/o Amino Acid and Ammonium Sulfate (Difco Laboratories, Inc.) and 5 g of ammonium sulfate dissolved in 100 ml of sterilized water, adjusted to about pH 5.2 and sterilized by filtration through a filter).
The yeast of the present invention is not particularly limited so far as it can produce γ-glutamylcysteine. Spe-

**EP 1 452 585 A1**

cifically, it includes yeasts belonging to the genus *Saccharomyces* such as *Saccharomyces cerevisiae,* yeasts belonging to the genus *Schizosaccharomyces* such as *Schizosaccharomyces pombe,* etc. It is preferred that the yeast strains of the present invention have polyploidy of diploidy or more in consideration of good growth.

Yeasts having diploidy or more polyploidy can be obtained by subjecting them to mutation treatment and selecting a strain that produces γ-glutamylcysteine, or by mating a monoploid yeast used for breeding a γ-glutamyl-cysteine-producing strain with a monoploid strain which harbors wild-type glutathione synthetase, allowing the obtained diploid yeast to form spores to thereby select a strain that has a reduced glutathione synthetase activity and produces γ-glutamylcysteine, and then mating two γ-glutamylcysteine-producing monoploid yeast strains having different mating types with each other. In a similar manner, yeasts having triploidy or more polyploidy can be obtained.

As described above, the yeast strain of the present invention has a resistance to MNNG and which produces γ-glutamylcysteine. Such a yeast strain does not lose the γ-glutamylcysteine synthetase activity but has a reduced glutathione synthetase activity. As a result, it accumulates γ-glutamylcysteine in the cells.

The term "having reduced glutathione synthetase activity" means that the glutathione synthetase activity of the yeast strain of the present invention is lower than that of the wild strain, including the case in which the activity is lost. However, it is preferred that the yeast strain of the present invention retains a glutathione synthetase activity lower than that of the wild strain rather than that the strain loses the glutathione synthetase activity.

The yeast strain as described above can be screened by the following steps.

(a) From a mass of yeast, yeast strains having a resistance to a certain concentration range of MNNG are selected, and

(b) From the selected strains, a strain having a reduced glutathione synthetase activity and γ-glutamylcysteine productivity is selected.

The yeast strain having a resistance to a certain concentration range of MNNG can be selected, for example, by culturing yeast in liquid medium or on agar medium containing various concentrations of MNNG and examining the growth thereof. The strain showing better growth than that of the wild strain has a resistance to MNNG. Alternatively, an agar medium having formed thereon a concentration gradient of MNNG may also be used for the selection. The concentration gradient of MNNG can be formed by placing on an agar medium a disk impregnated with an MNNG solution, for example, an MNNG solution in a concentration of 1 to 30 mg/ml. The farther from the disk, the lower the concentration of MNNG is in the medium. Therefore, inoculation of the mass of yeast that is the objective of selection on an agar medium and culturing after placing the disk in the center of the medium enables examination of a lot of yeasts for their resistance to various concentrations of MNNG. The inoculation amount of the yeast to be inoculated onto the medium is preferably such an amount that the appeared colonies will not contact each other and specifically about 100 to about 200 cells per plate is preferable.

The term "having a resistance to a certain concentration range of MNNG" means that the strain concerned grows better than the wild strain in the presence of a certain concentration of MNNG but cannot grow in the presence of MNNG in a concentration higher than that concentration. Thus, selection of a strain having not too high resistance to MNNG but a moderate resistance to MNNG enables efficient selection of a strain that does not lose γ-glutamyl-cysteine synthetase and has a reduced glutathione synthetase activity. The yeast strain having a resistance to a certain concentration range of MNNG forms a colony in a position at a certain distance from the disk on an agar medium on which a concentration gradient of MNNG is formed. The concentration of MNNG to which the yeast strain to be selected shows a resistance does not have to be identified but may be a relative value such as the distance from the disk on the agar medium.

The yeast strain having a resistance to a preferred concentration range of MNNG can be obtained by providing an previously selected yeast strain having a reduced glutathione synthetase activity and γ-glutamylcysteine productivity as a model strain and selecting a yeast strain having an MNNG resistance of the same level as that of the model strain. In the case where an agar medium on which a concentration gradient of MNNG has been formed is used, a strain of which the distance between the disk and the colony is of the same level as that of the model strain is selected. In this manner, use of a model strain as a control enables efficient selection of a yeast strain that is does not lose γ-glutamyl-cysteine synthetase activity and has a reduced glutathione synthetase activity as compared with the wild strain.

As the model strain, mention may be made of *Saccharomyces cerevisiae* Nα3 strain in which the glutathione synthetase encoded by the glutathione synthetase gene on the chromosome is deleted the C-terminal region consisting of the arginine residue at 370 and subsequent thereto. This strain has a reduced glutathione synthetase activity.

Furthermore, once a yeast strain that has a reduced glutathione synthetase activity as well as γ-glutamyl-cysteine productivity is obtained, selection can be performed without using any model strains by using as an index the concentration of MNNG showing the MNNG resistance of the strain.

The "mass of yeast" may be either a mass of yeast including a plurality of yeasts of different genera or species or a mass of yeasts of the same species including various mutants thereof. The yeast strain to be selected may be

5

either a strain that was subjected to artificial mutation treatment or a spontaneous mutant strain. The mass of yeast is prepared preferably by subjecting a wild-type strain of yeast or a mutant thereof having a suitable mutation as a parent strain to a mutation treatment. Alternatively, the mass of yeast may be a yeast transformant to which a glutathione synthetase gene having introduced therein a specific mutation or a random mutation has been introduced.

The yeast strain of the present invention can be obtained by combination of the mutation treatment with a screening step.

The mutation treatment includes a method in which tereatment with ultraviolet ray irradiation, or a mutagen usually used in mutation treatment such as MNNG, ethyl methansulfonate (EMS) or methyl methanesulfonate (MMS) is performed.

From the strains selected as described above, a strain that has a reduced glutathione synthetase activity and γ-glutamylcysteine productivity is selected. Also, it is preferred that the γ-glutamylcysteine synthetase activity of the selected strain be measured to confirm that the strain retains the activity of the enzyme. The γ-glutamylcysteine synthetase activity and glutathione synthetase activity can be measured by the method of Jackson (Jackson, R.C., Biochem. J., 111, 309 (1969)), and the method of Gushima et al. (Gushima, T. et al., J. Appl. Biochem., 5, 210 (1983)), respectively. Also, examination of the productivity of γ-glutamylcysteine enables confirmation that the γ-glutamylcysteine synthetase activity is retained and that the glutathione synthetase activity is reduced. Furthermore, examination of growth on a medium containing no glutathione enables confirmation as to whether the glutathione synthetase activity is reduced or lost.

The culture obtained by culturing the γ-glutamylcysteine-producing yeast strain obtained as described above under suitable conditions contains γ-glutamylcysteine. Such a culture or fractionation product thereof contains γ-glutamylcysteine. The culture may be a culture broth containing yeast cells or may be yeast cells collected therefrom, disrupted cells or cell extracts (yeast extracts). It is also recommendable to obtain a fraction containing γ-glutamylcysteine from the disrupted cells or yeast extracts.

Heating the above-mentioned culture containing γ-glutamylcysteine or a fraction thereof can release cysteine from γ-glutamylcysteine.

The medium to be used for culture is not particularly limited so far as it allows the yeast strain of the present invention to grow well and efficiently produce γ-glutamylcysteine. In particular, for a yeast strain that has a reduced glutathione synthetase activity but does not lose that activity can grow well on a medium containing no glutathione. Therefore, media usually used on an industrial scale can be used. Necessary nutrients may be added to the medium depending on the characteristics of the strain used, if necessary.

Culture conditions and preparation of yeast extracts and the like may be performed in the same manner as usual yeast culture and preparation of yeast extracts and the like are performed. The yeast extracts may be those obtained by treating hot water extract of the yeast cells or those obtained by treating digested yeast cells.

The above-mentioned culture or fraction thereof that contains γ-glutamylcysteine or cysteine can be used for the production of foods and beverages. The foods and beverages include alcoholic beverages, bread foods, and fermented food flavoring materials. Production of cysteine from γ-glutamylcysteine by heat treatment may be performed during the production of foods and beverages or after their production.

The above-mentioned foods and beverages are produced by mixing a culture or a fraction thereof containing γ-glutamylcysteine or cysteine with a food or beverage raw material and processing the mixture into a food or beverage. The food and beverage of the present invention can be produced by using the same raw materials as those for usual foods and beverages and by the same method as that for usual foods and beverages except that the above-mentioned culture or fractionation product is used. Such raw materials include, for example, rice, barley, cornstarch, etc. for alcoholic beverages, wheat flour, sugar, table salt, butter, fermentation yeast, etc., and soybean and wheat for bread foods, etc. for fermented food flavoring materials.

Brief explanation of the Drawings

Fig. 1 is a diagram construction of plasmid GSH2Mdash/pYES2dash containing a cassette for substituting a weakend-type glutathione synthetase gene.

Fig. 2 is a schematic diagram illustrating gene substitution of glutathione synthetase gene with the cassette shown in Fig. 1.

Description of Preferred Embodiments

Hereinafter, the present invention will be illustrated in more detail.

Example 1 Screening of a strain having a reduced glutathione synthetase using an MNNG resistance as a phenotype

<1> Breeding of Nα3 strain as a screening model strain

(1) Preparation of a cassette for substituting a weakened-type glutathione synthetase gene

From *Saccharomyces cerevisiae* isolated from nature, a monoploid Nα1 strain (uracil auxotroph) was obtained by a conventional method. Using Nα1 strain, a model strain Nα3 harboring a weakened-type glutathione synthetase gene was obtained by the method described below.

A region encoding about 120 base pairs from the midstream of an open reading frame (ORF) of the glutathione synthetase gene of *Saccharomyces cerevisiae* S2 strain to the downstream of the ORF was amplified by a polymerase chain reaction (PCR) using primer F1 (CAGATTCCGAGTTTACTGGA (SEQ ID NO:1)) and R1 (AGAAGGAATGAGCCTAAAACAGC (SEQ ID NO:2)) and Pyrobest DNA Polymerase (Takara Shuzo) under the conditions indicated by the manufacturer. The S2 strain was obtained as follows. Commercially available *Saccharomyces cerevisiae* for use in foods was allowed to form spores to obtain a monoploid baker's yeast S strain (MATα). Further, from the S strain was obtained an S2 strain, which is a uracil-requiring strain, by using SDFOA agar medium containing uracil (SD medium containing 2% purified agar, 50 mg/l of uracil and 1 g/l of 5-fluorooritic acid hydrate in final concentrations)

The gene fragment amplified as described above was purified and an enzymatic reaction was performed at 72°C for 10 minutes under the following conditions to add A to the termini.

| (composition of reaction mixture) | |
| --- | --- |
| Gene fragment solution | 5 μl |
| 10× PCR buffer (MgCl$_2$ free) | 5μl |
| 25 mM MgCl$_2$ | 3 μl |
| 2.5 mM dATP | 5 μl |
| Taq DNA polymerase (Takara Shuzo) | 0.5 μl |
| Purified water | 31.5 μl |
| Total | 50 μl |

The gene fragment obtained as described above was ligated to plasmid pGEM-T Easy (Promega Corporation) by a conventional method to obtain a plasmid GSH2dash/pGEM.

Next, a codon corresponding to the amino acid at a position 370 of the glutathione synthetase gene contained in GSH2dash/pGEM was substituted by a stop codon. This operation was performed by using QuikChange™ Site-Directed Mutagenesis Kit (Stratagene) according to the protocol provided by the manufacturer. As the primers were used GSH2M-F1 (GGCAGGGAAGGCAAGTAGCTGGCATTAAGTGAGCCCTC (SEQ ID NO:3)) and GSH2M-R1 (GAGGGCTCACTTAATGCCAGCTACTTGCCTTCCCTGCC (SEQ ID NO:4)). In this manner, a plasmid GSH2Mdash/pGEM was prepared.

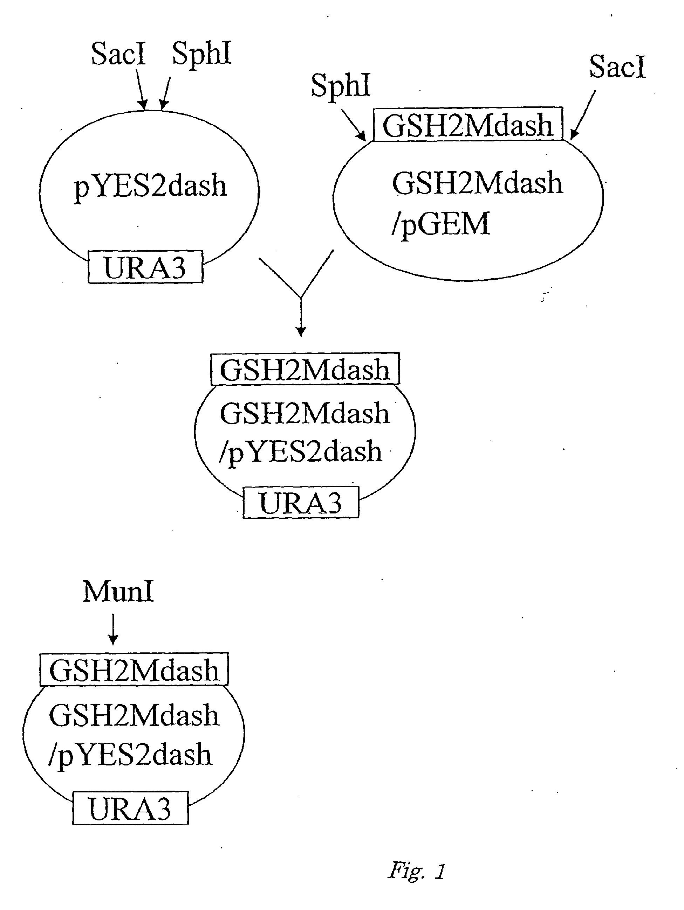

Separately, a plasmid corresponding to the plasmid pYES2 (Invitrogen) of which 2μ ori was removed was prepared. pYES2 was cleaved with restriction enzymes *Ssp*I and *Nhe*I and the cleaved ends were blunted and then ligated to obtain a plasmid pYES2dash. pYES2dash and GSH2Mdash/pGEM were each cleaved with restriction enzymes *Sac*I and *Sph*I to cleave out a fragment containing URA3 gene from pYES2dash and a glutathione synthetase gene fragment having a mutation from GSH2Mdash/pGEM, and these fragments were ligated. Thus, a plasmid GSH2Mdash/pYES2dash was prepared. GSH2Mdash/pYES2dash was digested with a restriction enzyme *Mun*I to obtain a cassette for gene substitution (Fig. 1).

(2) Construction of a strain having a weakened-type glutathione synthetase gene substitution

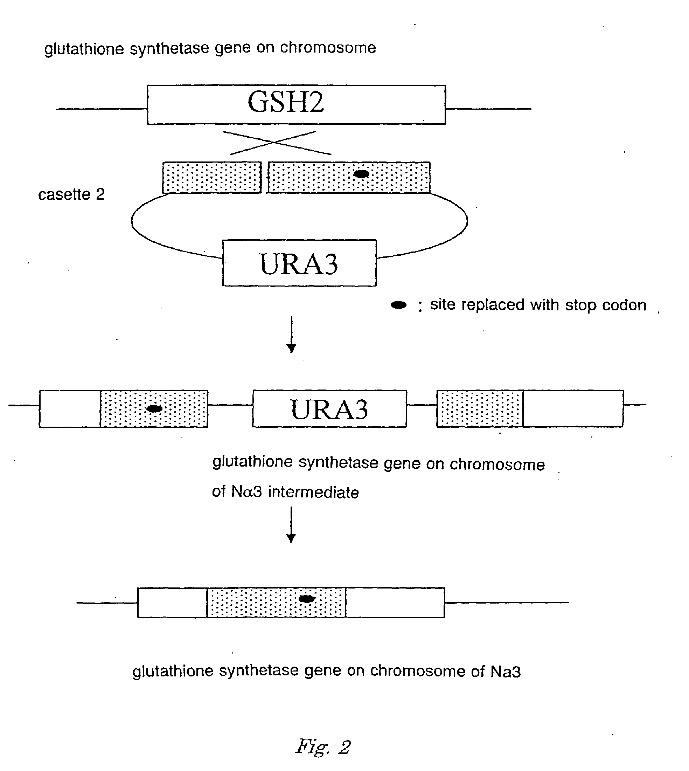

Gene substitution of the glutathione synthetase gene of the Nα1 strain was performed by using the cassette prepared as described above (Fig. 2). The Nα1 strain was precultured, and the culture was subcultured in 50 ml of YPD medium until the culture reached the logarithmic growth phase. The cultured cells were suspended in 1M sorbitol and mixed with the cassette, and then transformation was performed by electroporation. The transformant strains were cultured on an SD plate containing 1 mM glutathione and strains that grow thereon were selected. Incorporation of the cassette at the desired site on the chromosome was confirmed by PCR, and the obtained strain was designated as Nα3 intermediate strain.

Then, as shown in Fig. 2, the following operation was performed in order to leave only the weakened-type glutathione synthetase gene on the chromosome. The Nα3 intermediate was cultured on YPD medium and the cultured product was inoculated on an SDFOA plate containing 1 mM of glutathione. The sequence of the glutathione synthetase gene of a strain that grew on the plate was determined and it was confirmed that the sequence at the objective site was properly substituted. Thus, an Nα3 strain was obtained.

(3) Production of glutathione and γ-glutamylcysteine by Nα3 strain

The production amounts per unit time of γ-glutamylcysteine and glutathione in the logarithmic growth phase of Nα3 strain were investigated. After preculturing the Nα3 strain in YPD medium, the culture was inoculated in 50 ml of an SD medium (containing a necessary amount of uracil) and cultured with shaking at 30°C.

The production amounts of γ-glutamylcysteine and glutathione were measured as follows. That is, the culture was centrifuged to obtain cells, which were washed twice with distilled water and then subjected to hot water extraction treatment at 70°C for 10 minutes to obtain cell contents. This was centrifuged and the γ-glutamylcysteine and glutathione contents in the obtained supernatant were measured. On the other hand, yeast cells contained in a certain amount of medium were taken on a filter paper and heated at 105°C for 4 hours, followed by measurement of the weight of the remaining cells, which weight was defined as dry cell weight. Table 1 shows the contents of γ-glutamylcysteine and glutathione per dry cell weight.

Table 1

|  | Culture time until measurement | γ-glutamyl-cysteine (%) | glutathione (%) |
|---|---|---|---|
| Nα3 No.1 | Approximately 1.5 hours | 1.101 | 0.0043 |
| Nα3 No.2 | Approximately 3.8 hours | 1.117 | 0.0045 |

(4) Measurement of the degree of MNNG resistance of Nα3 strain

A filter was placed in the center of a YPD agar plate and a solution of 1 mg of MNNG dissolved in 30 μl of dimethyl sulfoxide (DMSO) was totally impregnated to the filter to prepare an MNNG concentration gradient agar medium in which the farther the position is from the central point, the less the MNNG concentration becomes. On this agar medium were spread the Nα1 strain and the Nα3 strain which had been cultured in YPD liquid media. After culturing at 30°C for 70 hours, the distances from the central point of the agar medium to the site where the yeasts formed colonies were measured. As a result, the distances were 2.3 cm for the Nα1 strain and 1.8 cm for the Nα3 strain.

<2> Screening of glutathione synthetase weakened strain

*Saccharomyces cerevisiae* used for foods was allowed to form spores by a conventional method to obtain a monoploid baker's yeast MS strain and mutation treatment was performed thereto using EMS as a mutagen. The mutation treatment was performed under the condition in which mortality was 90%.

The mutation treatment was performed as follows. That is, the MS strain was cultured with shaking at 30°C for 1 day in 50 ml of YPD medium and yeast cells were collected. The collected yeast cells were washed three times with 0.2 M sodium phosphate buffer (pH 7.5). Then, the yeast cells were suspended in a solution containing 9.2 ml of 0.2 M sodium phosphate buffer (pH 7.5), 0.5 ml of 40% D-glucose, and 0.3 ml of EMS (Nakarai Tesque, Code 155-19) and cultured with shaking at 30°C for 90 minutes. 10% Sodium thiosulfate (sterilized with a filter) (10 ml) was added to the culture to suspend the cells and then the suspension was left to stand at room temperature for 10 minutes to neutralize the mutagen. The yeast cells were collected and washed with 0.2 M sodium phosphate buffer (pH 7.5). Thus, mutation treatment of yeast was performed. On this occasion, the mortality was 90%.

The MS strain subjected to the mutation treatment as described above was spread on an MNNG concentration gradient agar medium (prepared by placing a filter in the center of a YPD agar plate and impregnating the filter with the whole amount of a solution of 1 mg of MNNG dissolved in 30 μl of DMSO) such that the number of appeared colonies becomes 200 or less, and subjected to standing culture at 30°C for 5 days. Yeast mutants that grew in an MNNG concentration range in which the yeast that produced an ordinary amount of glutathione (Nα1 strain) could not grow (about 2.3 cm from the central point of the agar medium) but the yeast that produced γ-glutamylcysteine but produced no or substantially no glutathione (Nα3 strain) could grow (about 1.8 cm from the central point of the agar medium) were isolated. Ninety-three (93) such mutant strains were measured for γ-glutamylcysteine productivity and a mutant strain AJ14809 of which the glutathione synthetase activity was reduced was obtained.

Example 2 Production of γ-glutamylcysteine by diploid yeast producing γ-glutamylcysteine

<1> Preparation of diploid yeast producing γ-glutamylcysteine

A monoploid yeast (MATα) AJ14809 strain, which was obtained by subjecting a monoploid yeast obtained by allowing commercially available baker's yeast (diploid) to form spores to a mutation treatment and selectionn utilizing an MNNG concentration gradient agar medium, was used as *Saccharomyces cerevisiae* with a reduced glutathione synthetase activity. This strain was selected as one having an MNNG resistance of the same level as that of the Nα3 strain by using the Nα3 strain as a control as described above.

The AJ14809 strain was deposited in National Institute of Advanced Industrial Science and Technology, International Patent Organism Depositary (Central 6, 1-1, Higashi 1-Chome, Tsukuba-shi, Ibaraki-ken, 305-8566, Japan) since October 1, 2001 under the accession number of FERM P-18546. The original deposit was converted to international deposit based on Budapest Treaty on December 1, 2002, and assigned the accession number of FERM BP-8228.

On the other hand, as a *Saccharomyces cerevisiae* wild strain, AJ14810 strain (MATa) was used. This strain has been deposited in National Institute of Advanced Industrial Science and Technology, International Patent Organism Depositary since October 1, 2001 under the accession number of FERM P-18547. The original deposit was converted to international deposit based on Budapest Treaty on December 1, 2002, and assigned the accession number of FERM BP-8229.

Each of the AJ14809 strain and AJ14810 strain was cultured in 4 ml of YPD test tube medium with shaking at 30°C. The cultures were mixed together in YPD test tube medium and cultured with shaking at 30°C. After confirming mating of the AJ14809 strain with the AJ14810 strain, the culture broth was spread on a YPD agar medium and cultured at 30°C. From among the yeasts that grew on the agar medium, a diploid strain having the glutathione synthetase gene derived from the AJ14809 strain and the glutathione synthetase gene derived from the AJ14810 strain was obtained. This diploid strain was allowed to form spores and 10 monoploid yeast strains were selected by a random spore method. The monoploid yeast strains which retained MATa and had reduced glutathione synthetase activity, and which contained 1% or more of γ-glutamylcysteine and accumulated 0.1% or less of glutathione per dry yeast cell in the logarithmic phase when cultured in an SD medium. These 10 strains were each mated with the AJ14809 strain to obtain a diploid yeast AJ14800 strain that had a reduced glutathione synthetase activity and that accumulated about 1.5% of γ-glutamylcysteine per dry yeast cell in the logarithmic phase when cultured in an SD medium.

As a control, a strain that produced a wild-type glutathione synthetase was selected from the diploid yeasts obtained by mating the AJ14809 strain with the AJ14810 strain and was designated as L strain.

<2> Measurement of degree of MNNG resistance of AJ14800 strain

The AJ14800 strain having a reduced glutathione synthetase activity and a diploid *Saccharomyces cerevisiae* L strain producing a wild-type glutathione synthetase were cultured in a YPD medium to obtain a culture broth, which was inoculated into a YPD medium containing 30 μg/ml or 60 μg/ml of MNNG or a YPD medium such that the absorbance at 660 nm became 0.1. The absorbance at 660 nm of the medium when subjected to standing culture at 30°C for 26 hours was measured to measure the degree of growth. As a result, as shown in Table 2, the glutathione synthetase weakened strain, AJ14800 strain, was more resistant to MNNG than the wild type glutathione synthetase retaining strain.

Table 2

|  | Degree of growth with 60 μg/ml of MNNG | Degree of growth with 60 μg/ml of MNNG |
|---|---|---|
| L | 0.0195 | 0.0139 |
| AJ14800 | 0.0540 | 0.0254 |

<3> Measurement of γ-glutamylcysteine accumulation amount of AJ14800 strain

After culturing the AJ14800 strain in a YPD medium for 40 hours, the cells were collected. The cells were inoculated into an SD medium such that the absorbance at 660 nm became 0.2. The intracellular γ-glutamylcysteine accumulation amount measured after 8 hours from the start of the culture was 1.50%.

Industrial Applicability

By the present invention, a yeast strain that has a reduced glutathione synthetase activity and produces γ-glutamylcysteine is provided. The strain of the present invention can be utilized for the production of γ-glutamylcysteine-containing foods or cysteine containing foods.

**Claims**

1.  A yeast strain which has a resistance to MNNG and which produces γ-glutamylcysteine.

2.  The yeast strain according to claim 1, wherein the strain has a degree of growth of 0.03 or more as calculated by the following formula:

    Degree of growth =

    $OD_{660}$ of the medium when cultured in MNNG-

    containing medium / $OD_{660}$ of the medium when cultured in

    MNNG-non-containing medium

when the yeast strain which has been precultured in a liquid medium is collected and inoculated to a YPD medium containing 30 µg/ml of MNNG (MNNG-containing medium) and a YPD medium containing no MNNG (MNNG-non-containing medium) so that the absorbance of each medium at 660 nm ($OD_{660}$) is 0.1, and then the strain is subjected to standing culture at a suitable temperature.

3. The yeast strain according to claim 1 or 2, wherein the strain has a reduced glutathione synthetase activity.

4. The yeast strain according to any one of claims 1 to 3, wherein when cultured in an SD medium, the γ-glutamyl-cysteine content per dry cell in logarithmic phase is 1% by weight or more.

5. The yeast strain according to any one of claims 1 to 4, wherein the strain has diploidy or more polyploidy.

6. The yeast strain according to any one of claims 1 to 5, wherein the strain belongs to the genus *Saccharomyces.*

7. A method of screening a yeast strain having a reduced glutathione activity and γ-glutamylcysteine productivity comprising the steps of:

(a) selecting yeast strains having a resistance to a certain concentration range of MNNG from a mass of yeast; and
(b) selecting a strain having a reduced glutathione synthetase activity and γ-glutamylcysteine productivity from among the selected strains.

8. The method according to claim 7, wherein in the step (a), the mass of yeast is prepared by subjecting a parent yeast strain to a mutation treatment.

9. The method according to claim 7 or 8, wherein in the step (a), the yeast strain having a resistance to a certain concentration range of MNNG is selected using an agar medium on which a concentration gradient of MNNG has been formed.

10. The method according to any one of claims 7 to 9, wherein in the step (a), a yeast model strain having a reduced glutathione synthetase activity and γ-glutamylcysteine productivity previously isolated is provided and a yeast strain having a resistance to MNNG of the same level as that of the model strain is selected.

11. A food or beverage comprising a culture obtained by culturing the yeast strain according to any one of claims 1 to 6 under a suitable condition, or a fractionation product of the culture containing γ-glutamylcysteine, or the culture or fractionation product in which cysteine has been produced by a heat treatment.

12. The food or beverage according to claim 11, wherein the food or beverage is an alcoholic beverage, a bread food, or a fermented food flavoring material.

13. A yeast extract produced by using a culture obtained by culturing the yeast strain according to any one of claims 1 to 6 under a suitable condition.

14. A method of producing food which contains γ-glutamylcysteine or cysteine, comprising the steps of culturing the yeast strain according to any one of claims 1 to 6, mixing the obtained culture or fractionation product thereof, or the culture or fractionation product thereof subjected to heat treatment with a beverage or food raw material, and processing the mixture into a food or beverage.

*Fig. 1*

glutathione synthetase gene on chromosome

*Fig. 2*

## INTERNATIONAL SEARCH REPORT

| International application No. |
| --- |
| PCT/JP02/12200 |

**A. CLASSIFICATION OF SUBJECT MATTER**
Int.Cl⁷ C12N1/16, C12Q1/04, A23L1/28, A23L1/305//(C12N1/16, C12R1:85)

According to International Patent Classification (IPC) or to both national classification and IPC

**B. FIELDS SEARCHED**

Minimum documentation searched (classification system followed by classification symbols)
Int.Cl⁷ C12N1/16, C12Q1/04, A23L1/28, A23L1/305

Documentation searched other than minimum documentation to the extent that such documents are included in the fields searched

Electronic data base consulted during the international search (name of data base and, where practicable, search terms used)
BIOSIS(DIALOG), WPI(DIALOG)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | Coblenz A. et al., Gcs1, a gene encoding gamma-glutamylcysteine synthetase in the fission yeast Schizosaccharomyces pombe. Yeast, 30 September, 1995 (30.09.95), Vol.11, No.12, pages 1171 to 1177 | 1-5,7-10<br>11-14<br>6 |
| Y<br>A | WO 00/30474 A1 (Ajinomoto Co., Inc.), 02 June, 2000 (02.06.00), & EP 1142493 A1 & JP 2000-583370 A | 11-14<br>1-10 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 24 January, 2003 (24.01.03) | 12 February, 2003 (12.02.03) |

| Name and mailing address of the ISA/<br>Japanese Patent Office | Authorized officer |
| --- | --- |
| Facsimile No. | Telephone No. |

Form PCT/ISA/210 (second sheet) (July 1998)